# EUROPEAN PATENT APPLICATION

(11) **EP 1 514 560 A1**
(43) Date of publication of application: **16.03.2005**
(21) Application number: 03736238.1
(22) Date of filing: 18.06.2003
(51) Int. Cl.: A61K 47/30, A61K 47/34, A61K 9/127, A61K 31/704, A61P 35/00

(54) **PROCESS FOR PRODUCING BLOCK COPOLYMER/DRUG COMPOSITE**

(30) Priority: 19.06.2002 JP 2002178619
(71) Applicant: NIPPON KAYAKU CO., LTD., Chiyoda-ku, Tokyo 102-8172 (JP); Japan Science and Technology Agency, Kawaguchi-shi, Saitama 332-0012 (JP); Sakurai, Yasuhisa, Suginamu-ku, Tokyo 168-0064 (JP)
(72) Inventor: NAKANISHI, Takeshi, Kamagaya-shi, Chiba 273-0137 (JP); SHIMIZU, Kazuhisa, Maebashi-shi, Gunma 371-0823 (JP)
(74) Representative: Wablat, Wolfgang, Dr.Dr.
(86) International application number: PCT/JP2003/007730
(87) International publication number: WO 2004/000362

(57) **Abstract**

A process for producing a block copolymer-drug composite that can be industrialized, wherein neither dialysis nor ultrafiltration is performed and wherein halogenated hydrocarbons are not used. The process for producing a block copolymer-drug composite is characterized by comprising the steps of dissolving an AB type block copolymer composed of hydrophilic polymer structure moiety and hydrophobic polyamino aid structure moiety together with a drug in water or a mixed solvent of water and a low-boiling-point organic solvent miscible with water and concentrating the resultant solution.

## Description

### Field of the Invention

The present invention relates to a process for producing a block copolymer-drug composite, comprising neither dialysis nor ultrafiltration process in obtaining a block copolymer-drug composite which contains a drug in a polymer micelle made of a block copolymer composed of hydrophilic polymer structure moiety and hydrophobic polyamino acid structure moiety.

### Background Art

A macromolecular block copolymer-drug composite obtained by allowing doxorubicin to be contained in a polymer micelle made of a block copolymer composed of polyethylene glycol structure moiety and polyaspartic acid derivative moiety is described in JP-A No. 7-69900 and the like, and it is described that this composite shows a higher antitumor effect within realm of low toxicity as compared with free doxorubicin. This block copolymer-drug composite is produced by dissolving a block copolymer and a drug in a mixed solvent of water and dimethylformamide (DMF: boiling point 153°C (101.3 kPa)), then, substituting an organic solvent dimethylformamide by water by a dialysis operation, further, purifying the mixture by dialysis and ultrafiltration operations. However, when dialysis and ultrafiltration operations are conducted, a part of contained drug (for example, doxorubicin) is also removed (Drug Delivery System, 16, No. 5, 401-408 (2001)), consequently, the drug is not used efficiently and the drug content cannot be increased for a block copolymer, further, the content varies continuously, therefore, it is also difficult to make the drug content constant. In addition, dialysis and ultrafiltration operations are both simple and convenient method for performance in a laboratory, however, a special equipment is necessary for industrial production, and these processes need a long period of time for actual production, and these are not practical production methods manifesting high industrial reality.

JP-A No. 2001-226294 describes a process of producing a macromolecular block copolymer-drug composite comprising the steps of emulsifying a chloroform solution of a block copolymer and a water-poorly-soluble drug by mechanical stirring, ultrasonic wave irradiation and the like and removing chloroform by evaporation, however, use of chloroform and the like in a factory is limited, namely, it is not an industrially practical method due to reasons including also use of a halogenated hydrocarbon immiscible with water.

### SUMMARY OF THE INVENTION

An industrially practicable process for producing a block copolymer-drug composite using neither dialysis nor ultrafiltration operations and using no halogenated hydrocarbon has not been known, a practical method for enhancing the drug content of a block copolymer has not been found, therefore, a practical and novel method for producing a block copolymer-drug composite has been desired.

The present inventors have intensively studied for solving the above-mentioned problem, and resultantly completed the present invention.

Namely, the present invention relates to
(1) A process for producing a block copolymer-drug composite, comprising the steps of dissolving an AB type block copolymer composed of hydrophilic polymer structure moiety and hydrophobic polyamino acid structure moiety together with a drug in water or a mixed solvent of water and a low-boiling-point organic solvent miscible with water, and concentrating the resultant solution;
(2) The process for producing a block copolymer-drug composite according to (1), wherein neither dialysis nor ultrafiltration process is included in the production process;
(3) The process for producing a block copolymer-drug composite according to (1) or (2), wherein the hydrophilic polymer structure moiety in the AB type block copolymer is a polyethylene oxide derivative, and the hydrophobic polyamino acid structure moiety in the AB type block copolymer is polyaspartic acid containing aspartic acid having a side chain carboxyl group bonded by an anthracycline-based anticancer agent;
(4) The process for producing a block copolymer-drug composite according to (1) or (2), wherein the hydrophilic polymer structure moiety in the AB type block copolymer is a polyethylene oxide derivative, and the hydrophobic polyamino acid structure moiety in the AB type block copolymer is polyglutamic acid containing glutamic acid having a side chain carboxyl group bonded by an anthracycline-based anticancer agent;
(5) The process for producing a block copolymer-drug composite according to (3) or (4), wherein the anthracycline-based anticancer agent bonded to a side chain carboxyl group on the hydrophobic polyamino acid structure moiety is doxorubicin;
(6) The process for producing a block copolymer-drug composite according to any one of (1) to (5), wherein the drug is an anthracycline-based anticancer agent;
(7) The process for producing a block copolymer-drug composite according to (6), wherein the anthracycline-based anticancer agent is doxorubicin or a salt thereof;
(8) A process for producing a lyophilization preparation containing a block copolymer-drug composite, comprising the steps of dissolving an AB type block copolymer composed of hydrophilic polymer structure moiety and hydrophobic polyamino acid structure moiety together with a drug in water or a mixed solvent of water and a low-boiling-point organic solvent miscible with water, concentrating the resultant solution, and further lyophilizing this:

### BEST MODE FOR CARRING OUT THE INVENTION

The process for producing a block copolymer-drug composite of the present invention is a method comprising the steps of dissolving an AB type block copolymer composed of hydrophilic polymer structure moiety and hydrophobic polyamino acid structure moiety together with a drug in water or a mixed solvent of water and a low-boiling-point organic solvent miscible with water, and concentrating the resultant solution, and conducting particularly neither dialysis nor ultrafiltration operation.

In the present invention, dialysis is an operation using a semi-permeable membrane for removing low molecular weight components (including organic solvent and salt) by diffusion based on difference in concentration. Ultrafiltration is an operation using an ultrafiltration membrane (fractional molecular weight: 5000 to 50000) for removing low molecular weight components in a block copolymer-drug composite while condensing a solution.

The drug used in the present invention is not particularly restricted, and anticancer agents are preferable since the composite of the present invention is excellent in tumor accumulation property based on blood vessel permeability, and various anticancer agents clinically used are mentioned, particularly preferable are anthracycline-based anticancer agents. Examples of the anthracycline-based anticancer agent include daunorubicin, doxorubicin, pirarubicin, acrarubicin, epirubicin, idarubicin and the like, and particularly preferable is doxorubicin.

The hydrophilic polymer structure moiety in the block copolymer composed of hydrophilic polymer structure moiety and hydrophobic polyamino acid structure moiety includes all of usually known polymer compounds showing hydrophilicity, and specific examples thereof include polyethylene oxide, polysaccharide, polyacrylic acid, polymethacrylic acid, polyvinylpyrrolidone, polyvinyl alcohol and the like, and derivatives thereof, and particularly preferable are polyethylene oxide derivatives. As the polyethylene oxide derivative, for example, a hydrophilic polymer structure moiety in the general formula (I) described later is specifically mentioned.

The hydrophobic polyamino acid structure moiety in the present invention is not particularly restricted providing it is selected from α-amino acids, β-amino acids and the like or their derivatives and the like, and preferable are polyamino acids containing an amino acid having a side chain carboxyl group bonded by an anthracycline-based anticancer agent. Specific examples of the polyamino acid include polyaspartic acid, polyglutamic acid and the like. As the anthracycline-based anticancer agent residue bonded to a side chain carboxyl group, resides of anthracycline-based anticancer agents mentioned as the drug used in the present invention described above are quoted, and particularly preferable as the hydrophobic polyamino acid is polyaspartic acid containing aspartic acid bonded by doxorubicin. The weight ratio of an anthracycline-based anticancer agent in a hydrophobic polyamino acid structure moiety in one molecule of block copolymer is preferably 20% to 70%, more preferably 25% to 60%.

The AB type block copolymer composed of hydrophilic polymer structure moiety and hydrophobic polyamino acid structure moiety used in the present invention may be that produced on the basis of methods described in JP-A Nos. 7-69900, 5-955 and the like. That is, one-end methoxy group one-end aminopropyl group polyethylene glycol and β-benzyl-L-aspartate-N-carboxylic anhydride or γ-benzyl-L-glutamate-N-carboxylic anhydride are reacted, the end amino group of the resulted block copolymer is acylated and the side chain benzyl ester is hydrolyzed with alkali, the resultant side chain free carboxylic acid and an anthracycline-based anticancer agent are condensed using a condensing agent and reaction auxiliary. The block copolymer produced by methods in the publications is estimated to have a structure of the following general formula (I) , for example.

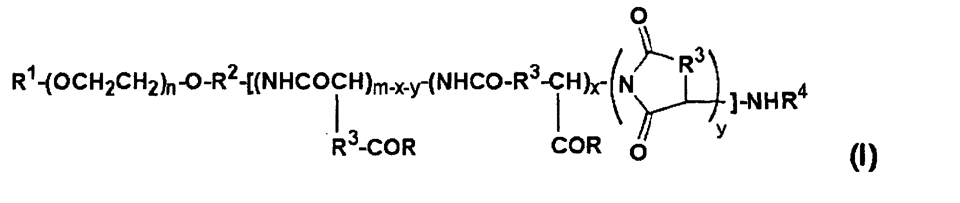

The order of bonding of constituent parts in polyamino acid structure moieties of the general formula (I) is not particularly restricted and may be random or regular. In the formula, R represents preferably a hydroxyl group or an anthracycline-based anticancer agent residue, R¹ represents preferably a hydrogen atom or a lower alkyl group, more preferably a lower alkyl group, R² represents preferably a lower alkylene group, R³ represents preferably a methylene group or an ethylene group, and R⁴ represents preferably a hydrogen atom or a lower acryl group, more preferably a lower acyl group. n represents preferably an integer of 5 to 1000, m represents preferably an integer of 2 to 300, x+y represents preferably an integer of 0 to 300, more preferably, n is 80 to 300, m is 20 to 50, x+y is 0 to 50, however, x+y is not larger than m. x and y can be any value including 0 providing the above-mentioned conditions are satisfied.

The anthracycline-based anticancer agent in an anthracycline-based anticancer agent residue represented by R is the same as the anthracycline-based anticancer agent mentioned as the drug used in the present invention described above, and the preferable anthracycline-based anticancer agent is also doxorubicin as described above.

The lower alkyl group represented by R¹ includes preferably straight chain or branched alkyl groups having 1 to 4 carbon atoms, specific examples thereof include a methyl group, an ethyl group, a n-propyl group, an i-propyl group, a n-butyl group, a t-butyl group and the like, and more preferably a methyl group.

The lower alkylene group represented by R² includes preferably straight chain or branched alkylene groups having 1 to 4 carbon atoms, specific examples thereof include a methylene group, an ethylene group, a trimethylene group, a 2-methyltrimethylene group, a tetramethylene group and the like, and more preferably a trimethylene group.

R³ represents preferably a methylene group or an ethylene group, more preferably a methylene group.

The lower acyl group represented by R⁴ includes preferably acyl groups having 1 to 4 carbon atoms, specific examples thereof include a formyl group, an acetyl group, a propionyl group, a butyryl group and the like, and more preferably an acetyl group.

The bonding mode of an anthracycline-based anticancer agent residue with a polyamino acid side chain in the general formula (I) is not particularly restricted, and preferably mentioned are amide bonds or ester bonds of an amino group or hydroxyl group of an anthracycline-based anticancer agent with a side chain carboxylic acid of a polyamino acid, particularly preferably are amide bonds of a primary amino group of an amino sugar moiety of an anthracycline-based anticancer agent with a carboxylic acid side chain of a polyamino acid.

Of block copolymers composed of hydrophilic polymer structure moiety and hydrophobic polyamino acid structure moiety of the general formula (I), particularly preferably mentioned are block copolymers in which R¹ is a methyl group, R² is a trimethylene group, R³ is a methylene group, R⁴ is an acetyl group, n is 80 to 300, m is 20 to 50, and x+y is 0 to 50 but not larger than m.

Next, the production process of the present invention will be illustrated. An AB type block copolymer composed of hydrophilic polymer structure moiety and hydrophobic polyamino acid structure moiety is dissolved in water or a mixed solvent of water and a low-boiling-point organic solvent miscible with water. As the low-boiling-point organic solvent in the present invention, organic solvents having a boiling point of 100°C (101.3 kPa) or less are mentioned, preferably are organic solvents having a boiling point of 50°C to 100°C (101.3 kPa) , specific examples of the low-boiling-point organic solvent miscible with water include methanol, ethanol, isopropanol, acetone and the like, and ethanol that can dissolve a block copolymer at high concentration is particularly preferable. The ratio of an organic solvent to water in a mixed solvent is not particularly restricted providing a block copolymer is dissolved, and desirably from 1:9 to 9:1, further desirably 1:3 to 3:1. The solvent may be heated for promoting dissolution. The temperature in heating may be in the range in which a block copolymer is not decomposed and stability is kept, and it is desirably from 30°C to 100°C, further desirably from 30°C to 60°C.

In the production process of the present invention, the concentration of a block copolymer in water or a mixed solvent of water and a low-boiling-point organic solvent miscible with water is not particularly restricted providing the copolymer is dissolved, and usually, it is preferably from 2% to 10%.

Subsequently, when a drug, for example, doxorubicin hydrochloride is added to the resultant block copolymer solution, it is dissolved to obtain a uniform solution. Further, pH of this solution may be controlled depending on stability and other requirements. The pH range in this case is not particularly restricted providing a drug is not decomposed, and in the case of doxorubicin hydrochloride, pH is preferably from 4 to 7.

If an organic solvent is contained in a solution, when the resultant composite solution is concentrated, this organic solvent, for example, ethanol is distilled off, to obtain a block copolymer-drug composite in which a drug is contained in an inner core of a polymer micelle made of a block copolymer. Therefore, it is not necessary to effect operations conduct conventional methods such as dialysis and ultrafiltration. In thus obtained block copolymer-drug composite, particle sizes of decades nm were confirmed and formation of a polymer micelle was clarified, by laser light scattering, and from quantification of a capsulated drug using a gel filtration column, it was confirmed that 80% or more of the drug used in production was capsulated.

Further, in the present invention, the resulted block copolymer-drug composite can be further lyophilized to obtain a lyophilized preparation. In lyophilization, salts having a buffering ability, for example, sodium phosphate, sodium acetate and the like may be added to make pH constant.

If this lyophilized preparation is, for example, re-dissolved in injection solvent, physiologic saline, physiologic saline endowed with a buffering ability, and the like, a block copolymer-drug composite solution is obtained.

In the present production process, a drug to be added is not lost in the course of production operation as in conventional production methods, consequently, a drug is not wasted, and additionally, the ratio of capsulation of a drug in a block copolymer can be enhanced. For instance, according to a production method shown in examples of JP-A No. 7-69900, the utilization ratio of doxorubicin used in production and the drug content ratio are as shown in Table 1, while in examples according to the present production method described later, the drug utilization ratio and the drug content ratio are both improved significantly as shown in Table 1.

**[Table 1]**

| | JP-A No. 7-69900 | | | | Present invention |
|---|---|---|---|---|---|
| | Example 1 | Example 2 | Example 3 | Example 4 | |
| Utilization ratio of doxorubicin | 87% | 85% | 72% | 60% | 95.70% |
| Drug content ratio of composite | 3.91% | 9.04% | 2.83% | 7.56% | 16.31% |

The feature of a block copolymer-drug composite is that a drug is contained in a micelle made of a block copolymer and a high antitumor effect is shown within realm of low toxicity as compared with the free drug, and increase of the drug capsulation ratio permits relative decrease in necessary amount of a block copolymer, consequently, the composite is practically excellent also from the standpoint of economy.

The following examples illustrate the present invention but do not limit the scope of the invention.

### Example 1

To 20.00 g of a block copolymer of the above-described general formula (I) wherein R¹ = methyl group, R² = trimethylene group, R³ = methylene group, R⁴ = acetyl group, R = hydroxyl group or doxorubicin residue (average ratio of doxorubicin bonding: 45%), the average molecular weight of polyethylene glycol is 5287 and the average unit number of aspartic acid is 28.1, was added 100 mL of injection solvent, the mixture was heated at 35°C to cause suspension. 6.0 mL of a 0.5 N sodium hydroxide solution was added to this and stirred, then, 100 mL of anhydrous ethanol was added. After confirmation of dissolving of the block copolymer, 3.906 g of doxorubicin hydrochloride was added and dissolved. 5.9 mL of 0.5 N sodium hydroxide was added to control pH to 6, and 188 mL of injection solvent was added. 1 hour after, the solution was filtrated through a membrane filter (Millipore; GV type 0.22 µm), then, a solvent was distilled off under reduced pressure to obtain a block copolymer-drug composite solution, and this was further lyophilized to obtain 22.96 g of a lyophilized substance of a block copolymer-drug composite. This composite had a drug content of 16.31%.

### Test Example 1

The lyophilized substance prepared in Example 1 was dissolved in phosphate buffer physiological saline (pH 7.4) to give a 1 mg/mL solution. This sample solution was allowed to stand still over night at 5°C, then, used for analysis. About 2 mL of Sephadex G-25 (manufactured by Farmacia: grade, fine) swollen with water was filled in a glass column. 200 µL of the sample solution was applied on this column. By developing with water, a portion included the micelle which capsulated the drug was removed, then diluted with dimethylformamide (DMF) of the same quantity, and then measured up to 5 mL using a water-DMF (1:1) solution (this is solution 1). Separately, 200 µL of the sample solution was placed in a 5 mL measuring flask, and measured up to 5 mL using a water-DMF (1:1) solution (this is solution 2). The solutions 1 and 2 were analyzed by a reversed phase HPLC decomposing a micelle structure, the ratio of the peak area of doxorubicin in the resulted chart (amount of free doxorubicin in solution 1/amount of free doxorubicin in solution 2) was calculated. The block copolymer-drug composite prepared in Example 1 had a drug content ratio of 94.9%. This result shows that a drug in the block copolymer-drug composite is contained in an inner core of a micelle made of the block copolymer.

### [Analytical condition of the reversed phase HPLC]

Column: Waters Symmetry C8 ϕ4.6 × 250 mm
Column temperature: 40°C
Eluant
(A) 0.05% MS-3 + 0.01% TFA (MS-3 is an ion pair reagent manufactured by GL Science, TFA is trifluoroacetic acid)
(B) CH₃CN

| Gradient | | | | | | | |
|---|---|---|---|---|---|---|---|
| Time (min) | 0 | 20 | 25 | 35 | 50 | 51 | 60 |
| B (%) | 20 | 40 | 40 | 90 | 90 | 20 | 20 |
| Detector | UV (485 nm) | | | | | | |
| Flow rate | 0.8 mL/min | | | | | | |
| Injection amount | 20 µL | | | | | | |

### Application Example 1

Colon 26 adenocarcinoma cells were transplanted subcutaneously in the subaxillary region of each CDF1 female mouse. When the volume of the tumor reached around 100 mm³, the block copolymer-drug composite obtained in Example 1 or doxorubicin hydrochloride was administered intravenously. The block copolymer-drug composite which had been lyophilized and stored in cool and dark place was dissolved with physiologic saline (pH 7.4) endowed with a buffering ability with phosphoric acid, and the dose in this case was determined base on the amount of a drug in the composite. The antitumor effect of the drug was judged from the number of mice showing disappearance of tumor (cured) 60 days after transplantation and the tumor proliferation inhibition ratio 14 days after administration. The results are shown in Table 2.

**[Table 2]**

| Effect on mouse colon cancer Colon 26 | | | | |
|---|---|---|---|---|
| Drug | dose (mg/kg) | T/C (%)^{a)} | Cure/n^{b)} | Tox./n^{c)} |
| Control group | 0.0 | 100 | 0/10 | 0/10 |
| Polymer-drug composition of Example 1 | 30.0 | 0.3 | 9/10 | 0/10 |
| | 24.0 | 0.8 | 9/10 | 0/10 |
| | 19.2 | 1.4 | 8/10 | 0/10 |
| Doxorubicin hydrochloride | 30.0 | 9.9 | 3/10 | 1/10 |
| | 24.0 | 6.4 | 4/10 | 0/10 |
| | 19.2 | 13.5 | 2/10 | 0/10 |

a): volume ratio of tumor against control groups 14 days after administration
b) : number of mice showing cure after 60 days observation period/number in one group
c) : number of mice died with toxicity/ number in one group

In comparison with a case of administration of doxorubicin hydrochloride itself, when the block copolymer-drug composite of the present invention is administered, a stronger tumor reducing effect was shown in all administration groups. The number of cured mice when observation period (60 days) was completed is larger than in a case of administration doxorubicin hydrochloride itself, and it was clarified that the block copolymer-drug composite shows a higher antitumor effect in a lower toxic range as compared with the free drug.

### Effect of the Invention

According to the process for producing a micelle type block copolymer-drug composite of the present invention, an operation property is significantly improved since neither dialysis process nor ultrafiltration process is contained, and the utilization ratio of a drug is excellent, the drug content can be enhanced and reproduced successfully, and the composite can be prepared in a shorter period of time as compared with conventional production processes. Since special equipments are not necessary and there is no necessity to use much water, this production process can be easily carried out and can be successfully industrialized. According to the present invention, a block copolymer-drug composite showing a higher antitumor effect in a lower toxicity range can be provided economically and simply.

## Claims

1. A process for producing a block copolymer-drug composite, comprising the steps of dissolving an AB type block copolymer composed of hydrophilic polymer structure moiety and hydrophobic polyamino acid structure moiety together with a drug in water or a mixed solvent of water and a low-boiling-point organic solvent miscible with water, and concentrating the resultant solution.

2. The process for producing a block copolymer-drug composite according to Claim 1, wherein neither dialysis nor ultrafiltration process is included in the production process.

3. The process for producing a block copolymer-drug composite according to Claim 1 or 2, wherein the hydrophilic polymer structure moiety in the AB type block copolymer is a polyethylene oxide derivative, and the hydrophobic polyamino acid structure moiety in the AB type block copolymer is polyaspartic acid containing aspartic acid having a side chain carboxyl group bonded by an anthracycline-based anticancer agent.

4. The process for producing a block copolymer-drug composite according to Claim 1 or 2, wherein the hydrophilic polymer structure moiety in the AB type block copolymer is a polyethylene oxide derivative, and the hydrophobic polyamino acid structure moiety in the AB type block copolymer is polyglutamic acid containing glutamic acid having a side chain carboxyl group bonded by an anthracycline-based anticancer agent.

5. The process for producing a block copolymer-drug composite according to Claim 3 or 4, wherein the anthracycline-based anticancer agent bonded to a side chain carboxyl group on the hydrophobic polyamino acid structure moiety is doxorubicin.

6. The process for producing a block copolymer-drug composite according to any one of Claims 1 to 5, wherein the drug is an anthracycline-based anticancer agent.

7. The process for producing a block copolymer-drug composite according to Claim 6, wherein the anthracycline-based anticancer agent is doxorubicin or a salt thereof.

8. A process for producing a lyophilization preparation containing a block copolymer-drug composite, comprising the steps of dissolving an AB type block copolymer composed of hydrophilic polymer structure moiety and hydrophobic polyamino acid structure moiety together with a drug in water or a mixed solvent of water and a low-boiling-point organic solvent miscible with water, concentrating the resultant solution, and further lyophilizing this.
